# EUROPÄISCHE PATENTSCHRIFT

(11) **EP 3 797 688 B1**
(45) Veröffentlichungstag und Bekanntmachung des Hinweises auf die Patenterteilung: **19.01.2022**
(21) Anmeldenummer: 20195978.0
(22) Anmeldetag: 14.09.2020
(51) Int. Cl.: A61B 5/274, H01R 13/627, H01R 13/633

(54) **KLEMMVORRICHTUNG UND SENSORKABEL**
CLAMPING DEVICE AND SENSOR CABLE
DISPOSITIF DE SERRAGE ET CÂBLE CAPTEUR

(30) Priorität: 27.09.2019 DE 102019006783
(43) Veröffentlichungstag der Anmeldung: 31.03.2021
(73) Patentinhaber: Drägerwerk AG & Co. KGaA, 23558 Lübeck (DE)
(72) Erfinder: Graßl, Thomas, 23558 Lübeck (DE)

(56) Entgegenhaltungen:
- US-B1- 6 487 430
- US-B1- 9 728 871

## Beschreibung

Die Erfindung betrifft eine Klemmvorrichtung zum elektrischen Koppeln eines Sensorkabels mit einer Elektrode. Ferner betrifft die Erfindung ein Sensorkabel zum elektrischen Koppeln einer Elektrode mit einem Diagnosegerät.

### STAND DER TECHNIK

Es ist bekannt, Sensorkabel über eine Klemmvorrichtung mit einer Elektrode elektrisch zu koppeln. Hierfür weisen herkömmliche Elektroden oftmals eine elektrisch leitende Niete auf, an welcher die Klemmvorrichtung anklemmbar ist. Ein anderes Ende des Sensorkabels ist zum elektrischen Koppeln mit einem Diagnosegerät, wie beispielsweise einem Patientenmonitor, einem EKG-Gerät, einem EEG-Gerät oder dergleichen, ausgebildet und weist hierfür beispielsweise einen mehrpoligen Stecker auf. Mittels des Diagnosegeräts ist beispielsweise eine Potenzialdifferenz zwischen zwei Elektroden detektierbar und für eine Bedienperson des Diagnosegeräts visualisierbar und/oder akustisch ausgebbar. Diagnosegeräte können beispielsweise ausgebildet sein, aus einer derartigen Potentialdifferenz in Abhängigkeit der Zeit und der Veränderung der Potentialdifferenz eine Herzfrequenz oder ST-Strecke zu bestimmen sowie optisch und/oder akustisch auszugeben.

Aus den Druckschriften US 9,226,680 B, DE 196 43 988 C1, US 4,040,697 A, US 6,397,439 B, DE 40 09 938 A1, US 2011 151 728 A und US 2004 203 273 A sind verschiedene gattungsgemäße Klemmvorrichtungen bekannt. Diese Klemmvorrichtungen weisen einen Klemmbereich mit zwei Klemmbacken und einen Betätigungsbereich mit zwei Betätigungsflächen auf. Durch Ausüben einer Druckkraft auf die Betätigungsflächen sind die Klemmbacken des Klemmbereichs auseinanderbewegbar, sodass die Klemmvorrichtung an der Elektrode platzierbar bzw. von dieser lösbar ist. Durch Abbau der Druckkraft auf die Betätigungsflächen sind die Klemmbacken wieder aufeinander zu bewegbar. Hierfür weisen die Klemmvorrichtungen eine Rückstellfeder bzw. einen Rückstellfederabschnitt auf. US 6 487 430 B1 offenbart eine Klemmvorrichtung gemäss dem Oberbegriff von Anspruch 1.

Herkömmliche Klemmvorrichtungen zum elektrischen Koppeln eines Sensorkabels mit einer Elektrode haben insbesondere den Nachteil, dass durch Verwinden des Sensorkabels ungünstige Kräfte und Momente auf die Elektrode und somit auf den Patienten übertragbar sind. Zudem haben herkömmliche Klemmvorrichtungen oftmals nur eine Vorzugsseite über welche sie mit der Elektrode koppelbar sind. Ein weiterer Nachteil vieler Klemmvorrichtungen ist, dass die Betätigungsflächen bei verhältnismäßig eng nebeneinander angeordneten Elektroden für die Bedienperson nur schwer zugänglich sind, sodass insbesondere beim Trennen der Klemmvorrichtung von der Elektrode häufig ein unbeabsichtigtes Kippmoment auf die Elektrode übertragen wird. Dies kann sich für den Patienten sehr unangenehm anfühlen und zum Ablösen der Elektrode führen. Dieses Problem tritt insbesondere bei kleinen Patienten, wie beispielsweise Kleinkindern, auf. Überdies haben viele Klemmvorrichtungen den Nachteil, dass diese Trennfugen aufweisen, welche nur sehr aufwendig desinfizierbar sind. Schließlich sind die meisten Klemmvorrichtung für die Verwendung bei sogenannten "Monoleads", also die Zusammenfassung mehrere Kabel zu einem Gesamtkabel, aufgrund der geometrischen Ausgestaltung nicht oder nur bedingt geeignet.

### OFFENBARUNG DER ERFINDUNG

Ausgehend von diesem Stand der Technik hat der Erfindung die Aufgabe zugrunde gelegen, eine Klemmvorrichtung sowie ein Sensorkabel bereitzustellen, die diese Nachteile nicht aufweisen oder zumindest teilweise nicht aufweisen. Es ist daher insbesondere die Aufgabe der vorliegenden Erfindung, eine Klemmvorrichtung zum elektrischen Koppeln eines Sensorkabels mit einer Elektrode sowie ein Sensorkabel zum elektrischen Koppeln einer Elektrode mit einem Diagnosegerät bereitzustellen, die mit einfachen Mitteln sowie auf eine kostengünstige Art und Weise ein einfaches elektrisches Koppeln sowie Entkoppeln von Klemmvorrichtung und Elektrode sicherstellen und dabei eine verbesserte Handhabung für die Bedienperson und/oder einen verbesserten Komfort für den Patienten gewährleisten.

Voranstehende Aufgabe wird durch eine Klemmvorrichtung mit den Merkmalen des unabhängigen Patentanspruchs 1 sowie durch ein Sensorkabel mit den Merkmalen des nebengeordneten Patentanspruchs 14 gelöst. Weitere Merkmale und Details der Erfindung ergeben sich aus den Unteransprüchen, der Beschreibung und den Zeichnungen. Dabei gelten Merkmale und Details, die im Zusammenhang mit der erfindungsgemäßen Klemmvorrichtung beschrieben sind, selbstverständlich auch im Zusammenhang mit dem erfindungsgemäßen Sensorkabel und jeweils umgekehrt, sodass bezüglich der Offenbarung zu den einzelnen Erfindungsaspekten stets wechselseitig Bezug genommen wird beziehungsweise werden kann.

Gemäß einem ersten Aspekt der Erfindung wird die Aufgabe durch eine Klemmvorrichtung zum elektrischen Koppeln eines Sensorkabels mit einem elektrischen Kontakt gelöst. Die Klemmvorrichtung weist einen umlaufenden, elastisch verformbaren Rahmen auf, wobei der Rahmen einen Rahmeninnenbereich, eine dem Rahmeninnenbereich abgewandte Rahmenaußenfläche, eine dem Rahmeninnenbereich zugewandte Rahmeninnenfläche, eine erste Rahmenstirnfläche und eine der ersten Rahmenstirnfläche entgegengesetzte zweite Rahmenstirnfläche aufweist. Ferner weist die Klemmvorrichtung eine am Rahmen gehaltene sowie sich in den Rahmeninnenbereich erstreckende Anlagevorrichtung zur Anlage an den elektrischen Kontakt sowie eine am Rahmen angeordnete Gegenhaltevorrichtung mit einem Gegenhalteelement auf. Die Anlagevorrichtung und die Gegenhaltevorrichtung sind ausgebildet, in einer Halteposition der Klemmvorrichtung einen Aufnahmeraum zur Aufnahme sowie zum Halten des elektrischen Kontakts zu bilden. Erfindungsgemäß ist die Klemmvorrichtung derart ausgebildet, dass durch ein beidseitiges Drücken gegen die Rahmenaußenfläche auf einer den Rahmeninnenbereich durchdringenden ersten Rahmenachse ein relatives Bewegen der Gegenhaltevorrichtung zur Anlagevorrichtung in eine erste Freigabeposition zum Freigeben des elektrischen Kontakts aus dem Aufnahmeraum bewirkbar ist. Zudem ist die Klemmvorrichtung derart ausgebildet, dass durch ein beidseitiges Drücken von außen gegen die Rahmenaußenfläche auf einer von der ersten Rahmenachse verschiedenen, den Rahmeninnenbereich durchdringenden zweiten Rahmenachse ein relatives Bewegen der Gegenhaltevorrichtung zur Anlagevorrichtung in eine von der ersten Freigabeposition verschiedene zweite Freigabeposition zum Freigeben des elektrischen Kontakts aus dem Aufnahmeraum bewirkbar ist.

Die Klemmvorrichtung ist vorzugsweise ausgebildet, in einem entspannten Zustand die Halteposition zu bilden. Ferner ist die Klemmvorrichtung zum elektrischen sowie mechanischen Koppeln mit einem Niet des elektrischen Kontakts ausgebildet. Der elektrische Kontakt kann beispielsweise als elektrischer Kontakt einer Elektrode ausgebildet sein, wie beispielsweise einer Elektrode, welche standardmäßig für EKG-Messungen bei Personen verwendet wird. Der im Rahmeninnenbereich von Anlagevorrichtung und Gegenhaltevorrichtung gebildete Aufnahmeraum ist ausgebildet, den Niet zumindest teilweise aufzunehmen. Der Aufnahmeraum wird beispielsweise derart von Anlagevorrichtung und Gegenhaltevorrichtung gebildet, dass der Niet in der Halteposition formschlüssig im Aufnahmeraum gehalten ist. Hierfür reichen drei voneinander beabstandete Haltepunkte, welche von der Anlagevorrichtung und Gegenhaltevorrichtung bereitgestellt sind. Weiter bevorzugt ist die Klemmvorrichtung ausgebildet, den Niet vollumfänglich oder zumindest teilumfänglich zu umgeben. Unter einem vollumfänglichen Umgeben wird im Rahmen der Erfindung ein Umgeben über mindestens 360° verstanden.

Besonders bevorzugt weist die Klemmvorrichtung keine engen Fugen, insbesondere zwischen relativ zueinander bewegbaren Teilen der Klemmvorrichtung auf. Eine enge Fuge ist beispielsweise eine Fuge zwischen zwei unmittelbar benachbarten bzw. sich berührenden Teilen. Eine Fuge mit einer Fugenbreite von mehr als 2 mm ist im Rahmen der Erfindung jedenfalls keine enge Fuge. Dies hat den Vorteil, dass die Klemmvorrichtung besonders leicht zu reinigen bzw. zu desinfizieren ist. Ein Infektionsrisiko für Patienten ist somit reduziert.

Die erste Rahmenachse schneidet vorzugsweise eine Rahmenmittelachse, welche durch einen Flächenmittelpunkt der ersten Rahmenstirnfläche und einen Flächenmittelpunkt der zweiten Rahmenstirnfläche verläuft. Somit sind Kraftangriffspunkte zum elastischen Zusammendrücken des Rahmens an entgegengesetzten Stellen an der Rahmenaußenfläche angeordnet. Vorzugsweise schneidet die erste Rahmenachse die Rahmenmittelachse in einer Mitte zwischen der ersten Rahmenstirnfläche und der zweiten Rahmenstirnfläche. Vorzugsweise verläuft die erste Rahmenachse parallel zur ersten Rahmenstirnfläche und/oder zweiten Rahmenstirnfläche. Die zweite Rahmenachse schneidet vorzugsweise die Rahmenmittelachse. Vorzugsweise schneidet die zweite Rahmenachse die Rahmenmittelachse in der Mitte zwischen der ersten Rahmenstirnfläche und der zweiten Rahmenstirnfläche. Vorzugsweise verläuft die zweite Rahmenachse parallel zur ersten Rahmenstirnfläche und/oder zweiten Rahmenstirnfläche. Vorzugsweise sind die erste Rahmenachse und die zweite Rahmenachse auf einer gemeinsamen Ebene angeordnet. Es ist erfindungsgemäß bevorzugt, dass ein Winkel zwischen der ersten Rahmenachse und der zweiten Rahmenachse zwischen 45° und 90° beträgt. Vorzugsweise beträgt dieser Winkel 90°.

Der Rahmen ist elastisch verformbar ausgebildet. Das bedeutet, dass durch ein Verformen des Rahmens durch Druckkräfte auf die Rahmenaußenfläche eine Rückstellkraft des Rahmens bewirkbar ist, wobei die Rückstellkraft ausgebildet ist, den Rahmen wieder in die ursprüngliche Form zurückzustellen. Die Klemmvorrichtung ist zum Halten des elektrischen Kontakts bzw. eines Niets des elektrischen Kontakts in der Halteposition, insbesondere bei entlastetem Rahmen, ausgebildet. Es kann erfindungsgemäß vorgesehen sein, dass in einem Teilbereich des Rahmeninnenbereichs ein Füllstoff, wie beispielsweise einen Schaumstoff, eine Netzstruktur oder dergleichen, angeordnet ist. Hierdurch kann eine Stabilität der Klemmvorrichtung verbessert werden.

Zum Halten des elektrischen Kontakts bzw. des Niets des elektrischen Kontakts weist die Klemmvorrichtung die Anlagevorrichtung sowie die Gegenhaltevorrichtung auf, welche sich in den Rahmeninnenbereich erstrecken.

Erfindungsgemäß weist die Gegenhaltevorrichtung ein Gegenhalteelement auf, welches relativ zur Anlagevorrichtung bewegbar, vorzugsweise verschwenkbar, ist. Im entlasteten Zustand des Rahmens ist der Niet zwischen der Anlagevorrichtung und dem Gegenhalteelement im Aufnahmeraum haltbar bzw. einklemmbar. Unter einem entlasteten Zustand wird im Rahmen der Erfindung insbesondere ein Zustand des Rahmens verstanden, bei welchem keine Druckkraft beidseitig gegen die Rahmenaußenseite wirkt. Gleichwohl kann der Rahmen im entlasteten Zustand eine elastische Spannung aufweisen, beispielsweise wenn ein Objekt, insbesondere ein Niet eines elektrischen Kontakts, im Aufnahmeraum angeordnet ist und somit die Anlagevorrichtung und die Gegenhaltevorrichtung kontaktiert. Vorzugsweise sind die Anlagevorrichtung und/oder das Gegenhalteelement ausgebildet, im entlasteten Zustand des Rahmens in eine Hinterschneidung des Niets einzugreifen.

Weiter bevorzugt sind keine weiteren Halteelemente vorgesehen, um ein Verformen der Klemmvorrichtung in die erste Freigabeposition sowie in die zweite Freigabeposition zum Lösen des Niets von der Klemmvorrichtung zu verbessern. Unter der ersten Freigabeposition bzw. der zweiten Freigabeposition der Klemmvorrichtung wird im Rahmen der Erfindung verstanden, dass die Anlagevorrichtung und die Gegenhaltevorrichtung, insbesondere das Gegenhalteelement, derart zueinander angeordnet sind, dass der Aufnahmeraum zumindest in eine Richtung zum Herausbewegen des Niets aus dem Aufnahmeraum geöffnet ist. Somit ist der Niet in der ersten Freigabeposition sowie in der zweiten Freigabeposition der Klemmvorrichtung leicht von dieser trennbar bzw. in diese einführbar.

Zum Lösen eines Niets von der Klemmvorrichtung bzw. zum elektrischen sowie mechanischen Koppeln der Klemmvorrichtung mit dem Niet gibt es erfindungsgemäß zwei Möglichkeiten. Durch beidseitiges Drücken auf die Rahmenaußenfläche auf der ersten Rahmenachse ist der Rahmen derart verformbar, dass die Anlagevorrichtung und die Gegenanlagevorrichtung relativ zueinander die erste Freigabeposition einnehmen. In der ersten Freigabeposition ist der Aufnahmeraum zur Freigabe des Niets in mindestens eine Richtung geöffnet, sodass der Niet von der Klemmvorrichtung lösbar ist. Durch beidseitiges Drücken auf die Rahmenaußenfläche auf der zweiten Rahmenachse ist der Rahmen derart verformbar, die Anlagevorrichtung und die Gegenanlagevorrichtung relativ zueinander die zweite Freigabeposition einnehmen, wobei die zweite Freigabeposition von der ersten Freigabeposition verschieden ist.

Eine erfindungsgemäße Klemmvorrichtung hat gegenüber herkömmlichen Klemmvorrichtungen den Vorteil, dass mit einfachen Mitteln sowie auf eine kostengünstige Art und Weise ein elektrisches sowie mechanisches Koppeln mit einem elektrischen Kontakt, insbesondere einem Niet eines elektrischen Kontakts, wie beispielsweise einer handelsüblichen EKG-Elektrode für Erwachsene oder Kinder, gewährleistet ist. Zudem hat die Klemmvorrichtung den großen Vorteil, dass durch beidseitiges Drücken auf die Rahmenaußenfläche auf unterschiedlichen Rahmenachsen der Klemmvorrichtung ein Öffnen der Klemmvorrichtung bewirkbar ist. Dies ist insbesondere bei eng nebeneinanderliegenden elektrischen Kontakten, insbesondere Elektroden, von Vorteil, wenn ein Angriffspunkt zum Öffnen der Klemmvorrichtung derart nah an einer benachbarten Klemmvorrichtung liegen, dass das Öffnen der Klemmvorrichtung durch die benachbarte Klemmvorrichtung behindert wird. Eine unbeabsichtigte Kollision mit anderen Klemmvorrichtungen ist somit leicht vermeidbar, indem zum Öffnen der Rahmen beispielsweise auf einer um 90° zur ersten Rahmenachse gedrehten zweiten Rahmenachse beidseitig zusammengedrückt wird.

Gemäß einer bevorzugten Weiterbildung der Erfindung kann bei einer Klemmvorrichtung vorgesehen sein, dass die Klemmvorrichtung derart ausgebildet ist, dass durch ein beidseitiges Drücken gegen die Rahmenaußenfläche auf die erste Rahmenachse ein Wegbewegen der Gegenhaltevorrichtung von der Anlagevorrichtung bewirkbar ist. Hierfür ist es bevorzugt, dass sich die Gegenhaltevorrichtung entlang der zweiten Rahmenachse erstreckt. Durch das beidseitige Drücken gegen den Rahmen ist der Rahmen vorzugsweise derart verformbar, dass sich die Druckpunkte annähern und um etwa 90° von den Druckpunkten beabstandete Rahmenpunkte voneinander entfernen. Die Gegenhaltevorrichtung ist vorzugsweise einem dieser Rahmenpunkte benachbart an dem Rahmen angeordnet, sodass der Abstand zwischen Gegenhaltevorrichtung und Anlagevorrichtung somit vergrößert wird. Dies hat den Vorteil, dass mit einfachen Mitteln sowie auf eine kostengünstige Art und Weise die erste Freigabeposition realisiert ist.

Vorzugsweise ist die Klemmvorrichtung derart ausgebildet, dass durch ein beidseitiges Drücken von außen gegen die Rahmenaußenfläche auf der zweiten Rahmenachse ein Wegbewegen des Gegenhalteelements von der zweiten Rahmenachse bewirkbar ist. Durch das beidseitige Drücken gegen den Rahmen ist der Rahmen vorzugsweise derart verformbar, dass sich die Druckpunkte annähern und um etwa 90° von den Druckpunkten beabstandete Rahmenpunkte voneinander entfernen. Die Gegenhaltevorrichtung ist vorzugsweise einem dieser Rahmenpunkte benachbart an dem Rahmen angeordnet, sodass hierdurch ein Wegschwenken der Gegenhaltevorrichtung bzw. des Gegenhalteelements, insbesondere im Zusammenspiel mit einem in dem Aufnahmeraum angeordneten Niet, bewirkbar ist. Dies hat den Vorteil, dass mit einfachen Mitteln sowie auf eine kostengünstige Art und Weise die zweite Freigabeposition realisiert ist.

Es ist bevorzugt, dass die Gegenhaltevorrichtung zwei Gegenhalteelemente aufweist, wobei die Klemmvorrichtung derart ausgebildet ist, dass durch ein beidseitiges Drücken gegen die Rahmenaußenfläche auf der zweiten Rahmenachse ein voneinander Wegbewegen der Gegenhalteelemente bewirkbar ist. Durch beidseitiges Drücken auf die Rahmenaußenfläche auf der zweiten Rahmenachse ist somit der Rahmen derart verformbar, dass ein Abstand der zwei Gegenhalteelemente voneinander vergrößert wird, insbesondere durch ein voneinander Wegschwenken der Gegenhalteelemente. Hierdurch wird die zweite Freigabeposition realisiert. Die Klemmvorrichtung ist vorzugsweise derart ausgebildet, das sich hierbei beide Gegenhalteelemente in entgegengesetzte Richtungen voneinander wegbewegen. Hierbei kann erfindungsgemäß vorgesehen sein, dass die Gegenhalteelemente unterschiedliche Bewegungen bezüglich Richtung und/oder Ausmaß durchführen. Alternativ kann die Klemmvorrichtung derart ausgebildet sein, dass sich bei einer solchen Betätigung nur ein Gegenhalteelement von dem anderen Gegenhalteelement wegbewegt, insbesondere wegschwenkt, wobei das andere Gegenhalteelement seine relative Lage zur Anlagevorrichtung nicht verändert. Erfindungsgemäß können noch weitere Gegenhalteelemente vorgesehen sein. Vorzugsweise ist die Klemmvorrichtung weiter ausgebildet, dass durch beidseitiges Drücken auf die Rahmenaußenfläche einer Rahmenachse, welche um bis zu ca. 30° zur zweiten Rahmenachse verdreht ist, die Gegenhalteelemente voneinander wegbewegbar, vorzugsweise verschwenkbar, sind. Zwei oder mehr Gegenhalteelemente haben den Vorteil, dass mit einfachen Mitteln sowie auf eine kostengünstige Art und Weise eine Haltekraft der Klemmvorrichtung erhöhbar sowie ein Lösen der Klemmvorrichtung erleichterbar sind.

Gemäß einer bevorzugten Weiterentwicklung der Erfindung kann bei einer Klemmvorrichtung vorgesehen sein, dass der Rahmen mehrere Gelenkbereiche aufweist, wobei die Gelenkbereiche ausgebildet sind nachzugeben, als Reaktion auf das beidseitige Drücken gegen die Rahmenaußenfläche auf der ersten Rahmenachse und/oder auf der zweiten Rahmenachse. Hierbei ist es bevorzugt, dass mindestens zwei Gelenkbereiche äquidistant zu der benachbarten ersten Rahmenachse oder zweiten Rahmenachse angeordnet sind. Vorzugsweise sind die Gelenkbereiche jeweils als Verjüngung des Rahmens ausgebildet. Die Verjüngung betrifft vorzugsweise einen Abstand zwischen der Rahmenaußenfläche und der Rahmeninnenfläche. Zusätzlich oder alternativ können die Gelenkbereiche auch aus einem anderen Werkstoff, insbesondere einem Werkstoff mit einer höheren Elastizität als zwischen den Gelenkbereichen ausgebildeten Rahmenbereiche, ausgebildet sein. Die zwischen den Gelenkbereichen ausgebildeten Rahmenbereiche können erfindungsgemäß auch unnachgiebig bzw. steif ausgebildet sein. Die erfindungsgemäße elastische Verformbarkeit des Rahmens wird in diesem Fall vorzugsweise über die Gelenkbereiche sichergestellt. Gelenkbereiche haben den Vorteil, dass ein steiferes Rahmenmaterial verwendbar ist bzw. dass ein Verformen des Rahmens zum Öffnen der Klemmvorrichtung durch beidseitiges Drücken auf die Rahmenaußenseite mit einfachen Mitteln sowie auf eine kostengünstige Art und Weise erleichtert ist.

Es ist erfindungsgemäß bevorzugt, dass die Anlagevorrichtung an einem der Gegenhaltevorrichtung zugewandten Anlageende eine Anlagemulde zur teilweise umschließenden Anlage des elektrischen Kontakts aufweist. Die Anlagemulde weist vorzugsweise eine Wölbung mit einem Innenradius auf, welcher einem Außenradius eines Niets eines handelsüblichen elektrischen Kontakts, insbesondere einer handelsüblichen Elektrode, wie beispielsweise einer EKG-Elektrode eines Erwachsenen oder eines Kindes, entspricht oder zumindest im Wesentlichen entspricht. Die Anlagemulde hat demnach von der ersten Rahmenstirnfläche aus betrachtet vorzugsweise eine konkave oder zumindest teilweise eine konkave Ausbildung. Eine derartige Anlagemulde hat insbesondere den Vorteil, dass mit einfachen Mitteln sowie auf eine kostengünstige Art und Weise ein besonders sicherer Halt der Klemmvorrichtung an dem elektrischen Kontakt gewährleistet ist.

Weiter bevorzugt ist an der Anlagevorrichtung ein Elektrokontakt zum elektrischen Koppeln der Klemmvorrichtung mit dem elektrischen Kontakt angeordnet. Alternativ oder zusätzlich kann auch an der Gegenhaltevorrichtung, insbesondere dem ersten Gegenhalteelement und/oder einem etwaigen zweiten Gegenhalteelement, ein Elektrokontakt angeordnet sein. Vorzugsweise ist der Elektrokontakt am Anlageende ausgebildet. Weiter bevorzugt bildet der Elektrokontakt das Anlageende oder zumindest einen Teil des Anlageendes. Der Elektrokontakt weist vorzugsweise eine Kontaktstärke auf, welche mindestens einer halben Rahmenstärke des Rahmens entspricht. Unter einer Rahmenstärke wird im Rahmen der Erfindung der Abstand zwischen der Rahmenaußenfläche und der Rahmeninnenfläche verstanden. Vorzugsweise weist der Elektrokontakt eine Kontaktstärke auf, welche mindestens einer halben Anlagestärke der Anlagevorrichtung entspricht. Der Elektrokontakt ist vorzugsweise derart ausgebildet und angeordnet, dass bei entlastetem Rahmen und an der Klemmvorrichtung gehaltenem elektrischen Kontakt ein elektrischer Kontakt zwischen dem Niet des elektrischen Kontakts und dem Elektrokontakt hergestellt ist. Dies hat den Vorteil, dass mit einfachen Mitteln sowie auf eine kostengünstige Art und Weise ein elektrischer Kontakt zwischen einem Sensorkabel und dem elektrischen Kontakt herstellbar ist.

In einer besonders bevorzugten Ausgestaltung der Erfindung kann bei einer Klemmvorrichtung vorgesehen sein, dass der Elektrokontakt derart ausgebildet und angeordnet ist, dass ein elektrisches Koppeln der Klemmvorrichtung mit dem elektrischen Kontakt sowohl über die erste Rahmenstirnfläche als auch die zweite Rahmenstirnfläche der Klemmvorrichtung erfolgen kann. Hierbei ist es bevorzugt, wenn der Elektrokontakt symmetrisch ausgebildet ist. Dies hat den Vorteil, dass mit einfachen Mitteln sowie auf eine kostengünstige Art und Weise ein Koppeln der Klemmvorrichtung mit dem elektrischen Kontakt vereinfacht ist, da die Kopplung über eine beliebige Rahmenstirnfläche der Klemmvorrichtung erfolgen kann. Ein Ausrichten der Klemmvorrichtung zum elektrischen Kontakt ist demnach erheblich vereinfacht, sodass das Koppeln durch die Bedienperson beispielsweise auch blind erfolgen kann.

Vorzugsweise weist die Rahmenaußenfläche zumindest teilweise eine rutschhemmende Oberfläche auf. Die rutschhemmende Oberfläche ist vorzugsweise zumindest in einem Schnittbereich der ersten Rahmenachse und/oder zweiten Rahmenachse mit der Rahmenaußenfläche ausgebildet. Ein rutschhemmendes Oberflächensegment weist vorzugsweise eine Segmentlänge in Umfangsrichtung des Rahmens von mindestens 1 cm auf. Alternativ kann die rutschhemmende Oberfläche auch vollumfänglich auf der Rahmenaußenseite angeordnet sein. Weiter bevorzugt ist die komplette Rahmenaußenseite als rutschhemmende Oberfläche ausgebildet. Die rutschhemmende Oberfläche weist vorzugsweise eine Aufrauhung, Riffel, Noppen oder dergleichen auf. Alternativ oder zusätzlich kann die rutschhemmende Oberfläche als Beschichtung ausgebildet sein oder eine Beschichtung aufweisen. Vorzugsweise weist die Rahmenaußenseite im Schnittbereich eine Markierung zum Visualisieren bevorzugter Druckpunkte auf. Die Visualisierung kann erfindungsgemäß auch als größere Rahmenstärke des Rahmens ausgebildet sein. Alternativ oder zusätzlich kann die Visualisierung als Graustufenkontrast und/oder farblicher Kontrast ausgebildet sein. Dies hat den Vorteil, dass mit einfachen Mitteln sowie auf eine kostengünstige Art und Weise die Bedienung der Klemmvorrichtung, insbesondere das Verformen des Rahmens durch beidseitiges Drücken, verbessert ist, da das Risiko des Abrutschens von den Druckpunkten reduziert und die bevorzugten Druckpunkte für die Bedienperson besser erkennbar sind.

Gemäß einer bevorzugten Ausführungsform der Erfindung ist mindestens eine der Rahmenstirnflächen planar und/oder gewölbt ausgebildet. Vorzugsweise sind beide Rahmenstirnflächen planar oder gewölbt ausgebildet. Die Wölbung ist vorzugsweise konvex ausgebildet. Weiter bevorzugt ist mindestens eine Kante, vorzugsweise sind beide Kanten zwischen Rahmenstirnflächen und der Rahmenaußenseite abgerundet. Die Oberfläche der Klemmvorrichtung, insbesondere der Rahmenstirnflächen, ist vorzugsweise im Wesentlichen glatt ausgebildet. Dies hat den Vorteil, dass mit einfachen Mitteln sowie auf eine kostengünstige Art und Weise eine Klemmvorrichtung bereitgestellt ist, die besonders leicht zu reinigen bzw. zu desinfizieren ist.

Vorzugsweise ist die Klemmvorrichtung einstückig aus einem Kunststoff gebildet. Vorzugsweise weist der Kunststoff federelastische Eigenschaften auf. Eine derartige Klemmvorrichtung ist mit einfachen Mitteln sowie auf eine kostengünstige Art und Weise herstellbar.

Besonders bevorzugt weist die Klemmvorrichtung eine Kabelschnittstelle zum elektrischen Koppeln der Klemmvorrichtung mit einem Kabel auf, wobei die Kabelschnittstelle zum bezogen auf den Rahmeninnenbereich seitlichen Führen des Kabels ausgebildet ist. Mit anderen Worten ist die Kabelschnittstelle ausgebildet, das Kabel gemäß einer Tangente bzw. einer tangentennahen Sekante zu führen. Für ein Sensorkabel bedeutet dies demnach, dass die Klemmvorrichtung seitlich vom Kabel des Sensorkabels absteht. Die Kabelschnittstelle ist vorzugsweise zum linearen Führen des Kabels ausgebildet. Dies hat den Vorteil, dass mit einfachen Mitteln sowie auf eine kostengünstige Art und Weise mittels der Klemmvorrichtung ein Sensorkabel herstellbar ist, welches eine Kopplung der Klemmvorrichtung mit einem elektrischen Kontakt zulässt bei welcher besonders geringe Biegemomente vom Kabel auf den elektrischen Kontakt übertragen werden.

Weiter bevorzugt weist die Klemmvorrichtung ein von einem Kunststoff umspritztes Federelement auf. Das Federelement ist vorzugsweise aus einem Federstahl oder dergleichen hergestellt. Eine derartige Klemmvorrichtung ist mit einfachen Mitteln sowie auf eine kostengünstige Art und Weise herstellbar und weist zudem verbesserte federelastische Eigenschaften auf.

Gemäß einem zweiten Aspekt der Erfindung wird die Aufgabe durch ein Sensorkabel zum elektrischen Koppeln eines elektrischen Kontakts mit einem Diagnosegerät gelöst. Das Sensorkabel weist ein mindestens einpoliges Kabel und mindestens eine Klemmvorrichtung zum elektrischen Koppeln des Sensorkabels mit dem elektrischen Kontakt auf. Erfindungsgemäß ist die mindestens eine Klemmvorrichtung als erfindungsgemäße Klemmvorrichtung ausgebildet.

Die Klemmvorrichtung ist vorzugsweise derart mit dem Kabel mechanisch gekoppelt, dass die Klemmvorrichtung seitlich vom Kabel absteht bzw. exzentrisch am Kabel gehalten ist. Überdies ist die Klemmvorrichtung vorzugsweise derart mit dem Kabel elektrisch gekoppelt, dass ein Elektrokontakt der Klemmvorrichtung zum elektrischen Koppeln der Klemmvorrichtung mit einem elektrischen Kontakt, insbesondere einem Niet eines elektrischen Kontakts, insbesondere einer Elektrode, elektrisch mit einem Leiter des Kabels gekoppelt ist.

Bei dem beschriebenen Sensorkabel ergeben sich sämtliche Vorteile, die bereits zu einer Klemmvorrichtung gemäß dem ersten Aspekt der Erfindung beschrieben worden sind. Demnach hat das erfindungsgemäße Sensorkabel gegenüber herkömmlichen Sensorkabeln den Vorteil, dass mit einfachen Mitteln sowie auf eine kostengünstige Art und Weise ein elektrisches sowie mechanisches Koppeln des Sensorkabels mit einem elektrischen Kontakt, insbesondere einem Niet einer Elektrode, wie beispielsweise einer handelsüblichen EKG-Elektrode für Erwachsene oder Kinder, gewährleistet ist. Zudem hat das Sensorkabel den großen Vorteil, dass durch beidseitiges Drücken auf die Rahmenaußenfläche auf unterschiedlichen Rahmenachsen der Klemmvorrichtung ein Öffnen der Klemmvorrichtung bewirkbar ist. Dies ist insbesondere bei eng nebeneinanderliegenden elektrischen Kontakten von Vorteil, wenn ein Angriffspunkt zum Öffnen der Klemmvorrichtung derart nah an einer benachbarten Klemmvorrichtung liegen, sodass das Öffnen der Klemmvorrichtung durch die benachbarte Klemmvorrichtung behindert wird. Eine unbeabsichtigte Kollision mit anderen Klemmvorrichtungen ist somit leicht vermeidbar, indem zum Öffnen der Rahmen beispielsweise auf einer um 90° zur ersten Rahmenachse gedrehten zweiten Rahmenachse beidseitig zusammengedrückt wird.

Es ist bevorzugt, dass das Kabel mehrpolig mit mehreren Leitern ausgebildet ist und dass das Sensorkabel mehrere Klemmvorrichtungen aufweist, wobei die Klemmvorrichtungen jeweils mit einem unterschiedlichen Leiter des Kabels elektrisch gekoppelt sind. Die Klemmvorrichtungen sind vorzugsweise derart mit dem Kabel mechanisch gekoppelt, dass die Klemmvorrichtungen seitlich vom Kabel abstehen bzw. exzentrisch am Kabel gehalten sind. Überdies sind die Klemmvorrichtungen vorzugsweise derart jeweils mit einem eigenen Leiter des Kabels elektrisch gekoppelt, dass ein Elektrokontakt der Klemmvorrichtung zum elektrischen Koppeln der Klemmvorrichtung mit einem elektrischen Kontakt, insbesondere einem Niet der Elektrode, elektrisch mit dem jeweiligen Leiter des Kabels gekoppelt ist. Dies hat den Vorteil, dass mit einfachen Mitteln sowie auf eine kostengünstige Art und Weise eine Mehrzahl von elektrischen Kontakten mit dem Sensorkabel koppelbar sind.

Besonders bevorzugt ist die mindestens eine Klemmvorrichtung, insbesondere wasserdicht und/oder formschlüssig, an das Kabel angebunden, insbesondere angegossen. Hierbei ist es bevorzugt, wenn zwischen der mindestens einen Klemmvorrichtung und dem Kabel keine Trennfugen ausgebildet sind. Dies hat den Vorteil, dass mit einfachen Mitteln sowie auf eine kostengünstige Art und Weise ein leicht zu reinigendes bzw. leicht desinfizierbares Sensorkabel bereitgestellt ist und somit das Infektionsrisiko bei Patienten reduziert ist.

### BEVORZUGTE AUSFÜHRUNGSBEISPIELE

Weitere, die Erfindung verbessernde Maßnahmen ergeben sich aus der nachfolgenden Beschreibung zu einigen Ausführungsbeispielen der Erfindung, welche in den Figuren dargestellt sind. Sämtliche aus den Ansprüchen, der Beschreibung oder der Zeichnung hervorgehende Merkmale und/oder Vorteile, einschließlich konstruktiver Einzelheiten und räumlicher Anordnungen können sowohl für sich, als auch in den verschiedenen Kombinationen erfindungswesentlich sein. Es zeigen jeweils schematisch:
- Figur 1: eine perspektivische Ansicht einer bevorzugten ersten Ausführungsform einer erfindungsgemäßen Klemmvorrichtung,
- Figur 2: eine Draufsicht der Klemmvorrichtung aus Figur 1,
- Figur 3: eine perspektivische Ansicht einer bevorzugten zweiten Ausführungsform einer erfindungsgemäßen Klemmvorrichtung,
- Figur 4: eine perspektivische Ansicht einer bevorzugten dritten Ausführungsform einer erfindungsgemäßen Klemmvorrichtung,
- Figur 5: eine perspektivische Ansicht einer bevorzugten vierten Ausführungsform einer erfindungsgemäßen Klemmvorrichtung,
- Figur 6: eine Draufsicht einer bevorzugten ersten Ausführungsform eines erfindungsgemäßen Sensorkabels, und
- Figur 7: eine Draufsicht einer bevorzugten zweiten Ausführungsform eines erfindungsgemäßen Sensorkabels.

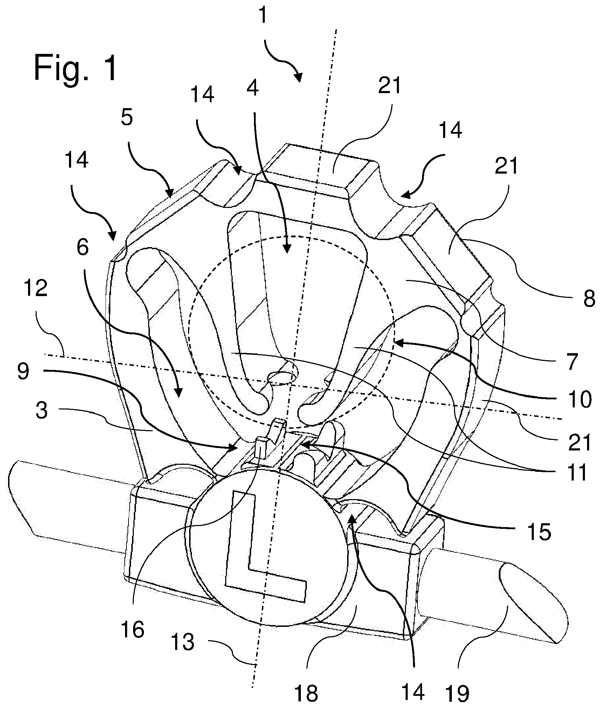

Elemente mit gleicher Funktion und Wirkungsweise sind in den Fig. 1 bis 7 jeweils mit denselben Bezugszeichen versehen.

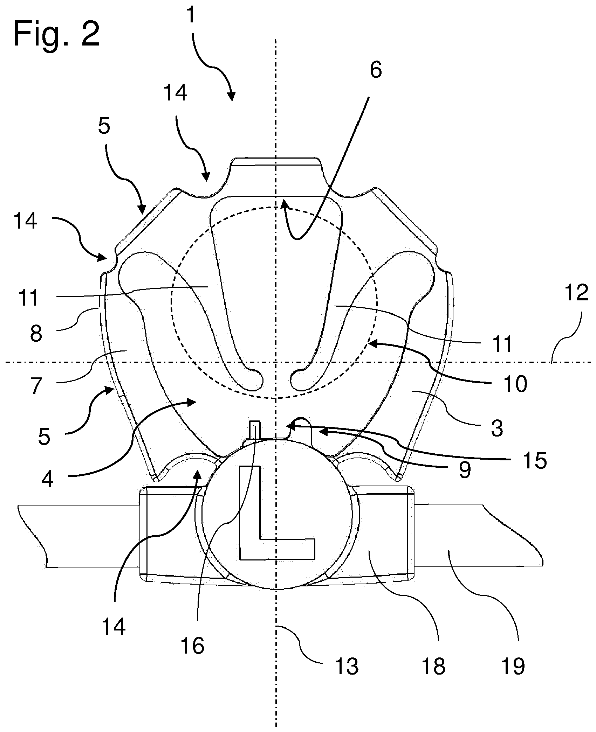

In Fig. 1 und Fig. 2 ist eine bevorzugte erste Ausführungsform einer erfindungsgemäßen Klemmvorrichtung 1 schematisch in einer perspektivischen Ansicht dargestellt. Die Klemmvorrichtung 1 weist einen umlaufenden Rahmen 3 auf, der einen Rahmeninnenbereich 4 vollumfänglich umschließt. Der Rahmen 3 weist eine dem Rahmeninnenbereich 4 abgewandte Rahmenaußenfläche 5, eine dem Rahmeninnenbereich zugewandte Rahmeninnenfläche 6 sowie eine sichtbare, zwischen der Rahmenaußenfläche 5 und der Rahmeninnenfläche 6 ausgebildete erste Rahmenstirnfläche 7 und eine in dieser Darstellung nicht sichtbare, der ersten Rahmenstirnfläche 7 entgegengesetzte zweite Rahmenstirnfläche 8 auf.

Der Rahmen 3 weist mehrere über den Rahmen 3 verteilte Gelenkbereiche 14 auf. Die Gelenkbereiche 14 sind in diesem bevorzugten ersten Ausführungsbeispiel als Verjüngungen des Rahmens 3 sowie als konkaver Verlauf der Rahmenaußenfläche 5 ausgebildet. Zwischen den Gelenkbereichen 14 ausgebildete Bereiche der Rahmenaußenfläche 5 sind als Angriffsflächen 21 für eine Bedienperson zum beidseitigen Zusammendrücken des Rahmens 3 ausgebildet. An einer in diesen Darstellungen unteren Seite des Rahmens 3 ist eine Kabelschnittstelle 18 im Rahmen 4 ausgebildet. Über die Kabelschnittstelle 18 ist die Klemmvorrichtung 1 mit einem Kabel 19 elektrisch und mechanisch gekoppelt. Die Kabelschnittstelle 18 ist derart ausgebildet, dass das Kabel 19 im Wesentlichen tangential zum Rahmen 3 geführt ist.

Auf einer dem Rahmeninnenbereich 4 zugewandten Seite der Kabelschnittstelle 18 ist eine Anlagevorrichtung 9 zur Anlage an einen nicht dargestellten Niet eines nicht dargestellten elektrischen Kontakts ausgebildet. Die Anlagevorrichtung 9 weist zur Aufnahme eines Teilbereichs des Niets eine Anlagemulde 15 auf. Ein Bereich der Anlagemulde 15 ist als Elektrokontakt 16 zum Herstellen einer elektrischen Kopplung zwischen dem Niet und dem Kabel 19 ausgebildet. An einer der Anlagefläche 9 gegenüberliegenden Seite des Rahmens 3 ist eine Gegenhaltevorrichtung 10 angeordnet. Die Gegenhaltevorrichtung 10 weist zwei voneinander beabstandete Gegenhalteelemente 11 auf, welche sich zueinander konvergierend in den Rahmeninnenbereich 4 erstrecken. Der Anlagevorrichtung 9 benachbarte Endbereiche der Gegenhalteelemente 11 sind zur Anlage an den Niet des elektrischen Kontakts ausgebildet. Die Klemmvorrichtung 1 ist in einem entspannten Zustand dargestellt, also in einem Haltezustand zum Halten des Niets des elektrischen Kontakts.

In den Fig. 1 und Fig. 2 sind beispielhaft eine erste Rahmenachse 12 und eine um 90° zur ersten Rahmenachse 12 angeordnete zweite Rahmenachse 13 dargestellt. Die erste Rahmenachse 12 und die zweite Rahmenachse 13 spannen eine gemeinsame Fläche auf, welche parallel zur ersten Rahmenstirnfläche 7 bzw. zweiten Rahmenstirnfläche 8 sowie zwischen der ersten Rahmenstirnfläche 7 und der zweiten Rahmenstirnfläche 8 angeordnet ist. Durch beidseitiges Drücken gegen die Angriffsflächen 21 auf der ersten Rahmenachse 12 sind der in dieser Darstellung linke und rechte Rahmenteil des Rahmens 3 aufeinander zu bewegbar. Dies hat zur Folge, dass sich der in dieser Darstellung obere und untere Rahmenteil des Rahmens voneinander wegbewegen. Somit wird hierdurch bewirkt, dass sich die Anlagevorrichtung 9 und Gegenhaltevorrichtung 10 voneinander wegbewegen, sodass der Niet des elektrischen Kontakts freigebbar ist. Durch beidseitiges Drücken gegen die Angriffsflächen 21 auf der zweiten Rahmenachse 13 sind der in dieser Darstellung obere und untere Rahmenteil des Rahmens 3 aufeinander zu bewegbar. Dies hat zur Folge, dass die beiden Gegenhalteelemente 11 voneinander weggeschwenkt werden, sodass der Niet des elektrischen Kontakts freigebbar ist.

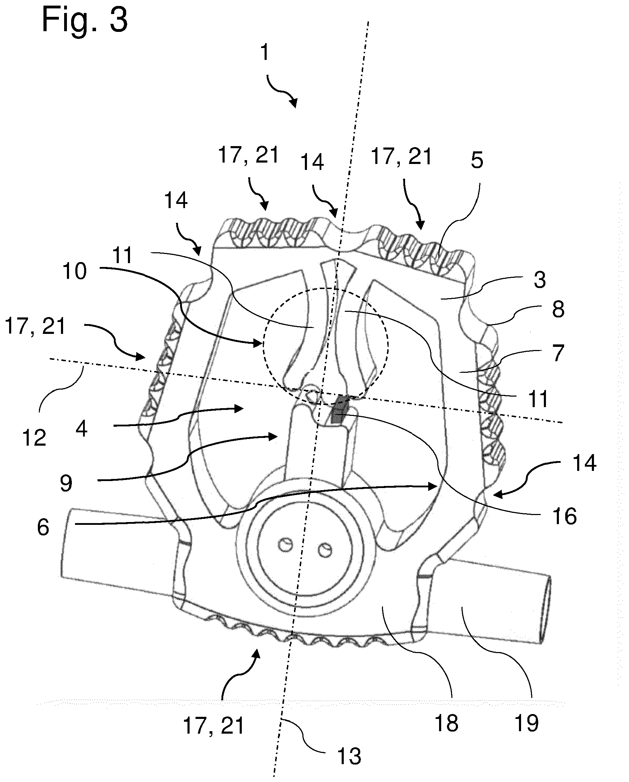

In Fig. 3 ist eine bevorzugte zweite Ausführungsform einer erfindungsgemäßen Klemmvorrichtung 1 schematisch in einer perspektivischen Ansicht dargestellt. Die Klemmvorrichtung 1 unterscheidet sich von der Klemmvorrichtung 1 gemäß der ersten Ausführungsform insbesondere in einer Anbindung der Gegenhalteelemente 11 an den Rahmen 3. Zudem ist ein Gelenkbereich 14 auf der zweiten Rahmenachse 13 angeordnet. Schließlich weisen die Angriffsflächen 21 der Rahmenaußenfläche 5 eine rutschhemmende Oberfläche 17 auf.

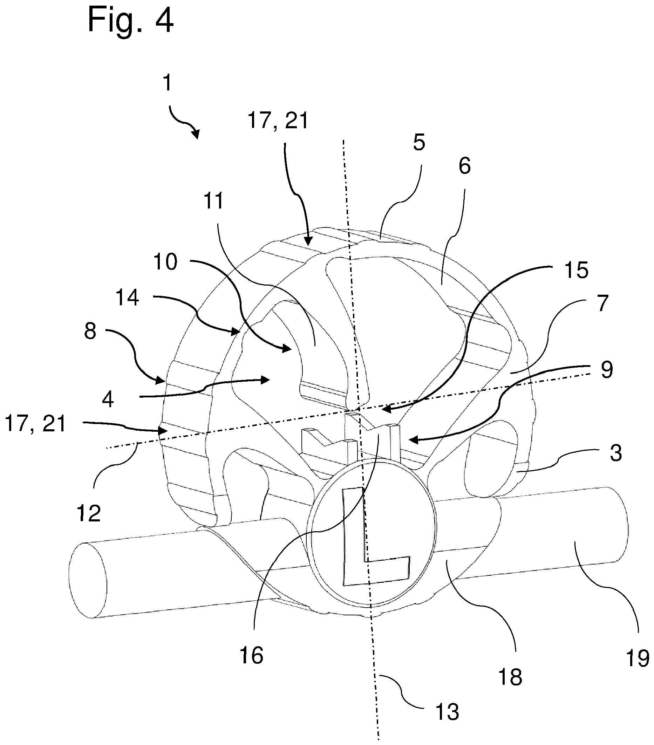

Fig. 4 zeigt eine bevorzugte dritte Ausführungsform einer erfindungsgemäßen Klemmvorrichtung 1 schematisch in einer perspektivischen Ansicht. Die Klemmvorrichtung 1 gemäß der dritten Ausführungsform unterscheidet sich von den Klemmvorrichtungen 1 der Fig. 1 bis 3 insbesondere dadurch, dass die Gegenhaltevorrichtung 10 nur ein Gegenhalteelement 11 aufweist. Das Gegenhalteelement 11 - und somit die gesamte Gegenhaltevorrichtung 10 - ist schräg zur Anlagevorrichtung 9 angeordnet. In einer alternativen Ausführungsform kann das Gegenhalteelement 11 auch direkt auf die Anlagevorrichtung 9 weisen oder mit der Anlagevorrichtung 9 in einer gemeinsamen Flucht angeordnet sein. In dem gezeigten Zustand ist der Rahmen 3 entspannt, sodass ein Niet eines elektrischen Kontakts in der Klemmvorrichtung 1 einklemmbar ist. Durch beidseitiges Drücken gegen die Angriffsflächen 21 auf der zweiten Rahmenachse 13 sind der in dieser Darstellung obere und untere Rahmenteil des Rahmens 3 aufeinander zu bewegbar. Dies hat zur Folge, dass das Gegenhalteelement 11 von der Anlagevorrichtung 9 - in dieser Ansicht im Uhrzeigersinn - weggeschwenkt wird, sodass der Niet des elektrischen Kontakts freigebbar ist.

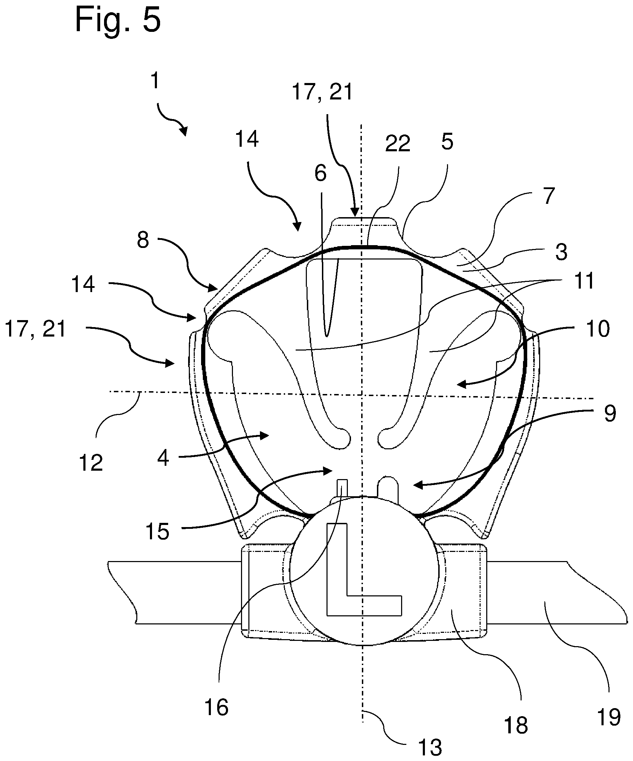

In Fig. 5 ist eine bevorzugte vierte Ausführungsform einer erfindungsgemäßen Klemmvorrichtung 1 schematisch in einer Draufsicht abgebildet. Die Klemmvorrichtung 1 gemäß der vierten Ausführungsform unterscheidet sich von den Klemmvorrichtungen 1 der Fig. 1 bis 3 insbesondere dadurch, dass der Rahmen ein etwa ringförmiges Federelement 22 aufweist, welches vorzugsweise vom Rahmenwerkstoff umgossen bzw. umspritzt ist. Demnach ist es bevorzugt, dass das Federelement 22 zwischen der Rahmenaußenfläche 5, der Rahmeninnenfläche 6, der ersten Rahmenstirnfläche 7 und der zweiten Rahmenstirnfläche 8 des Rahmens 3 angeordnet ist. Das Federelement 22 ist ausgebildet, den Rahmen 3 nach einer Verformung in den abgebildeten entspannten Zustand zurückzustellen, wobei in dem entspannten Zustand des Rahmens 3 ein Niet von der Klemmvorrichtung 1 einklemmbar ist. Im Bereich der Kabelschnittstelle 19 ist das Federelement 22 unterbrochen. Vorzugsweise ist das Federelement 22 an der Kabelschnittstelle 19 gehalten bzw. an dieser fixiert. Das Federelement 22 besteht vorzugsweise aus Federstahl oder weist vorzugsweise zumindest Federstahl auf.

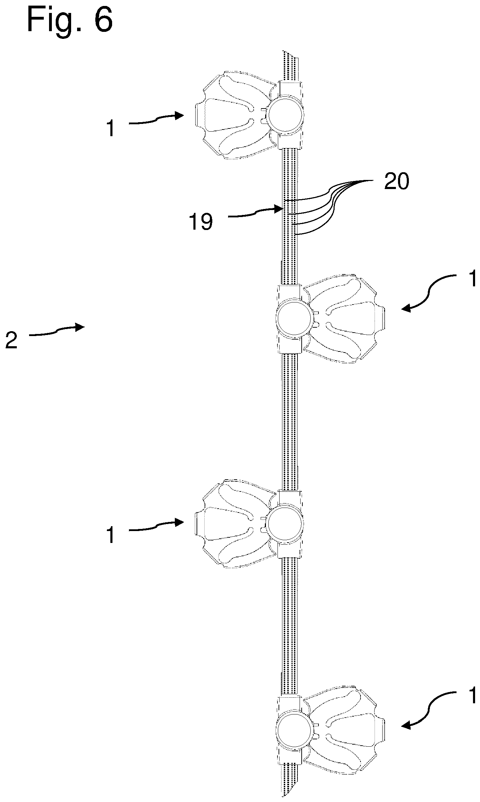

Fig. 6 zeigt eine bevorzugte erste Ausführungsform eines erfindungsgemäßen Sensorkabels 2 schematisch in einer Draufsicht. In dieser Ansicht ist nur ein Ausschnitt des Sensorkabels 2 dargestellt. Vorzugsweise weist das Sensorkabel 2 an einem Ende einen nicht abgebildeten Stecker zum elektrischen Koppeln mit einem Analysegerät auf. Das Sensorkabel 2 weist ein Kabel 19 mit einer Vielzahl von elektrisch voneinander isolierten einzelnen Leitern 20 auf. Voneinander beabstandet sind mehrere erfindungsgemäße Klemmvorrichtungen 1 an dem Kabel 19 derart angeordnet, dass diese seitlich vom Kabel 19 abstehen. In diesem Ausführungsbeispiel stehen die Klemmvorrichtungen 1 auf zwei entgegengesetzten Seiten vom Kabel 19 ab. Es ist bevorzugt, dass die Klemmvorrichtungen 1 derart an dem Kabel 19 gehalten sind, dass zwischen den Klemmvorrichtungen 1 und dem Kabel 19 keine Trennfugen vorhanden sind. Dies hat den Vorteil, dass das Sensorkabel 2 somit leichter desinfizierbar ist. Jede Klemmvorrichtung 1 ist mit einem anderen Leiter 20 des Kabels 19 elektrisch gekoppelt.

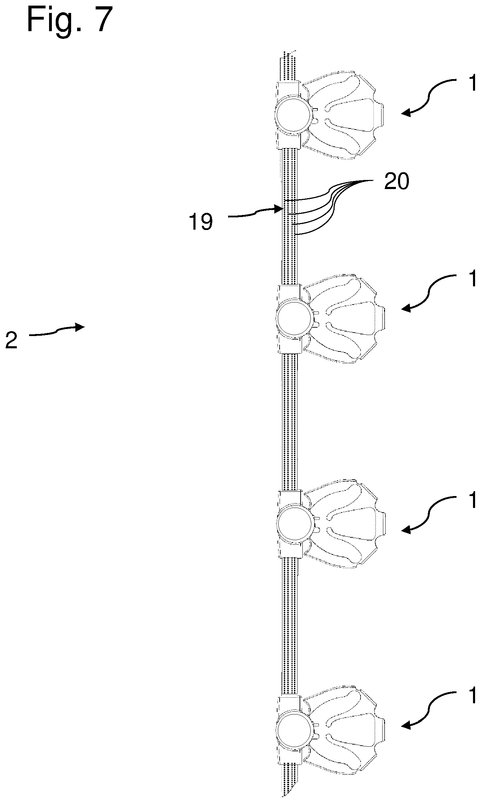

In Fig. 7 ist eine bevorzugte zweite Ausführungsform eines erfindungsgemäßen Sensorkabels 2 schematisch in einer Draufsicht gezeigt. Die bevorzugte zweite Ausführungsform des erfindungsgemäßen Sensorkabels 2 unterscheidet sich von der in Fig. 6 abgebildeten ersten Ausführungsform insbesondere in dem Merkmal, dass die Klemmvorrichtungen 1 allesamt auf derselben Seite vom Kabel 19 abstehen.

### BEZUGSZEICHENLISTE

- 1: Klemmvorrichtung
- 2: Sensorkabel
- 3: Rahmen
- 4: Rahmeninnenbereich
- 5: Rahmenaußenfläche
- 6: Rahmeninnenfläche
- 7: erste Rahmenstirnfläche
- 8: zweite Rahmenstirnfläche
- 9: Anlagevorrichtung
- 10: Gegenhaltevorrichtung
- 11: Gegenhalteelement
- 12: erste Rahmenachse
- 13: zweite Rahmenachse
- 14: Gelenkbereich
- 15: Anlagemulde
- 16: Elektrokontakt
- 17: rutschhemmende Oberfläche
- 18: Kabelschnittstelle
- 19: Kabel
- 20: Leiter
- 21: Angriffsfläche
- 22: Federelement

## Patentansprüche

1. Klemmvorrichtung (1) zum elektrischen Koppeln eines Sensorkabels (2) mit einem elektrischen Kontakt, aufweisend einen umlaufenden, elastisch verformbaren Rahmen (3), wobei der Rahmen (3) einen Rahmeninnenbereich (4), eine dem Rahmeninnenbereich (4) abgewandte Rahmenaußenfläche (5), eine dem Rahmeninnenbereich (4) zugewandte Rahmeninnenfläche (6), eine erste Rahmenstirnfläche (7) und eine der ersten Rahmenstirnfläche (7) entgegengesetzte zweite Rahmenstirnfläche (8) aufweist, eine am Rahmen (3) gehaltene sowie sich in den Rahmeninnenbereich (4) erstreckende Anlagevorrichtung (9) sowie eine am Rahmen (3) angeordnete Gegenhaltevorrichtung (10) mit einem Gegenhalteelement (11), wobei die Anlagevorrichtung (9) und die Gegenhaltevorrichtung (10) ausgebildet sind, in einer Halteposition der Klemmvorrichtung (1) einen Aufnahmeraum zur Aufnahme sowie zum Halten des elektrischen Kontakts zu bilden,
**dadurch gekennzeichnet, dass** die Klemmvorrichtung (1) derart ausgebildet ist, dass durch ein beidseitiges Drücken gegen die Rahmenaußenfläche (5) auf einer den Rahmeninnenbereich (4) durchdringenden ersten Rahmenachse (12) ein relatives Bewegen der Gegenhaltevorrichtung (10) zur Anlagevorrichtung (9) in eine erste Freigabeposition zum Freigeben des elektrischen Kontakts aus dem Aufnahmeraum bewirkbar ist, und dass durch ein beidseitiges Drücken von außen gegen die Rahmenaußenfläche (5) auf einer von der ersten Rahmenachse (12) verschiedenen, den Rahmeninnenbereich (4) durchdringenden zweiten Rahmenachse (13) ein relatives Bewegen der Gegenhaltevorrichtung (10) zur Anlagevorrichtung (9) in eine von der ersten Freigabeposition verschiedene zweite Freigabeposition zum Freigeben des elektrischen Kontakts aus dem Aufnahmeraum bewirkbar ist.

2. Klemmvorrichtung (1) nach Anspruch 1,
**dadurch gekennzeichnet, dass** die Klemmvorrichtung (1) derart ausgebildet ist, dass durch ein beidseitiges Drücken gegen die Rahmenaußenfläche (5) auf die erste Rahmenachse (12) ein Wegbewegen der Gegenhaltevorrichtung (10) von der Anlagevorrichtung (9) bewirkbar ist.

3. Klemmvorrichtung (1) nach Anspruch 1 oder 2,
**dadurch gekennzeichnet, dass** die Klemmvorrichtung (1) derart ausgebildet ist, dass durch ein beidseitiges Drücken von außen gegen die Rahmenaußenfläche (5) auf der zweiten Rahmenachse (13) ein Wegbewegen des Gegenhalteelements (11) von der zweiten Rahmenachse (13) bewirkbar ist.

4. Klemmvorrichtung (1) nach einem der vorangehenden Ansprüche,
**dadurch gekennzeichnet, dass** die Gegenhaltevorrichtung (10) zwei Gegenhalteelemente (11) aufweist, wobei die Klemmvorrichtung (1) derart ausgebildet ist, dass durch ein beidseitiges Drücken gegen die Rahmenaußenfläche (5) auf der zweiten Rahmenachse (13) ein voneinander Wegbewegen der Gegenhalteelemente (11) bewirkbar ist.

5. Klemmvorrichtung (1) nach einem der vorangehenden Ansprüche,
**dadurch gekennzeichnet, dass** der Rahmen (3) mehrere Gelenkbereiche (14) aufweist, wobei die Gelenkbereiche (14) ausgebildet sind nachzugeben, als Reaktion auf das beidseitige Drücken gegen die Rahmenaußenfläche (5) auf der ersten Rahmenachse (12) und/oder auf der zweiten Rahmenachse (13).

6. Klemmvorrichtung (1) nach einem der vorangehenden Ansprüche,
**dadurch gekennzeichnet, dass** die Anlagevorrichtung (9) an einem der Gegenhaltevorrichtung (10) zugewandten Anlageende eine Anlagemulde (15) zur teilweise umschließenden Anlage des elektrischen Kontakts aufweist.

7. Klemmvorrichtung (1) nach einem der vorangehenden Ansprüche,
**dadurch gekennzeichnet, dass** an der Anlagevorrichtung (9) ein Elektrokontakt (16) zum elektrischen Koppeln der Klemmvorrichtung (1) mit dem elektrischen Kontakt angeordnet ist.

8. Klemmvorrichtung (1) nach Anspruch 7,
**dadurch gekennzeichnet, dass** der Elektrokontakt (16) derart ausgebildet und angeordnet ist, dass ein elektrisches Koppeln der Klemmvorrichtung (1) mit dem elektrischen Kontakt sowohl über die erste Rahmenstirnfläche als auch die zweite Rahmenstirnfläche der Klemmvorrichtung (1) erfolgen kann.

9. Klemmvorrichtung (1) nach einem der vorangehenden Ansprüche,
**dadurch gekennzeichnet, dass** die Rahmenaußenfläche (5) zumindest teilweise eine rutschhemmende Oberfläche (17) aufweist.

10. Klemmvorrichtung (1) nach einem der vorangehenden Ansprüche,
**dadurch gekennzeichnet, dass** mindestens eine der Rahmenstirnflächen (7, 8) planar und/oder gewölbt ausgebildet ist.

11. Klemmvorrichtung (1) nach einem der vorangehenden Ansprüche,
**dadurch gekennzeichnet, dass** die Klemmvorrichtung (1) eine Kabelschnittstelle (18) zum elektrischen Koppeln der Klemmvorrichtung (1) mit einem Kabel (19) aufweist, wobei die Kabelschnittstelle (18) zum bezogen auf den Rahmeninnenbereich (4) seitlichen Führen des Kabels (19) ausgebildet ist.

12. Klemmvorrichtung (1) nach einem der vorangehenden Ansprüche,
**dadurch gekennzeichnet, dass** die Klemmvorrichtung (1) einstückig aus einem Kunststoff besteht.

13. Klemmvorrichtung (1) nach einem der vorangehenden Ansprüche,
**dadurch gekennzeichnet, dass** die Klemmvorrichtung (1) ein von einem Kunststoff umspritztes Federelement (22) aufweist.

14. Sensorkabel (2) zum elektrischen Koppeln eines elektrischen Kontakts mit einem Diagnosegerät, aufweisend ein mindestens einpoliges Kabel (19) und mindestens eine Klemmvorrichtung (1) zum elektrischen Koppeln des Sensorkabels (2) mit dem elektrischen Kontakt,
**dadurch gekennzeichnet, dass** die mindestens eine Klemmvorrichtung (1) als Klemmvorrichtung (1) nach einem der vorangehenden Ansprüche ausgebildet ist.

15. Sensorkabel (2) nach Anspruch 14,
**dadurch gekennzeichnet, dass** das Kabel (19) mehrpolig mit mehreren Leitern (20) ausgebildet ist und dass das Sensorkabel mehrere Klemmvorrichtungen (1) aufweist, wobei die Klemmvorrichtungen (1) jeweils mit einem unterschiedlichen Leiter (20) des Kabels (19) elektrisch gekoppelt sind.

16. Sensorkabel (2) nach Anspruch 14 oder 15,
**dadurch gekennzeichnet, dass** die mindestens eine Klemmvorrichtung (1) an das Kabel (19) angegossen ist.

## Claims

1. Clamping device (1) for electrically coupling a sensor cable (2) to an electrical contact, having an encircling, elastically deformable frame (3), wherein the frame (3) has a frame interior region (4), a frame exterior face (5) that faces away from the frame interior region (4), a frame interior face (6) that faces the frame interior region (4), a first frame end face (7) and a second frame end face (8) that is opposite the first frame end face (7), a bearing device (9) that is held on the frame (3) and extends into the frame interior region (4), as well as a counter holding device (10) that is disposed on the frame (3) and has a counter holding element (11), wherein the bearing device (9) and the counter holding device (10) in a holding position of the clamping device (1) are configured to form a receptacle space for receiving and holding the electrical contact,
**characterized in that**
the clamping device (1) is configured in such a manner that a relative movement of the counter holding device (10) in relation to the bearing device (9) to a first releasing position for releasing the electrical contact from the receptacle space is able to be effected by pressing on both sides of the frame exterior face (5) on a first frame axis (12) that penetrates the frame interior region (4), and **in that** a relative movement of the counter holding device (10) in relation to the bearing device (9) to a second releasing position, different from the first releasing position, for releasing the electrical contact from the receptacle space is able to be effected by pressing from the outside on both sides of the frame exterior face (5) on a second frame axis (13), different from the first frame axis (12), that penetrates the frame interior region (4).

2. Clamping device (1) according to Claim 1, **characterized in that** the clamping device (1) is configured in such a manner that a movement of the counter holding device (10) away from the bearing device (9) is able to be effected by pressing on both sides of the frame exterior face (5) onto the first frame axis (12).

3. Clamping device (1) according to Claim 1 or 2, **characterized in that** the clamping device (1) is configured in such a manner that a movement of the counter holding element (11) away from the second frame axis (13) is able to be effected by pressing from the outside on both sides on the frame exterior face (5) on the second frame axis (13).

4. Clamping device (1) according to one of the preceding claims, **characterized in that** the counter holding device (10) has two counter holding elements (11), wherein the clamping device (1) is configured in such a manner that a diverging movement of the counter holding elements (11) is able to be effected by pressing on both sides on the frame exterior face (5) on the second frame axis (13).

5. Clamping device (1) according to one of the preceding claims, **characterized in that** the frame (3) has a plurality of articulated regions (14), wherein the articulated regions (14) are configured so as to yield in response to the compression on both sides on the frame exterior face (5) on the first frame axis (12) and/or on the second frame axis (13).

6. Clamping device (1) according to one of the preceding claims, **characterized in that** the bearing device (9) on a bearing end that faces the counter holding device (10) has a bearing depression (15) for bearing the electrical contact in a partially enclosing manner.

7. Clamping device (1) according to one of the preceding claims, **characterized in that** a contact pin (16) for electrically coupling the clamping device (1) to the electrical contact is disposed on the bearing device (9).

8. Clamping device (1) according to Claim 7, **characterized in that** the contact pin (16) is configured and disposed in such a manner that electrically coupling the clamping device (1) to the electrical contact can take place both by way of the first frame end face as well as the second frame end face of the clamping device (1).

9. Clamping device (1) according to one of the preceding claims, **characterized in that** the frame exterior face (5) at least in part has an anti-slip surface (17).

10. Clamping device (1) according to one of the preceding claims, **characterized in that** at least one of the frame end faces (7, 8) is configured so as to be planar and/or curved.

11. Clamping device (1) according to one of the preceding claims, **characterized in that** the clamping device (1) has a cable interface (18) for electrically coupling the clamping device (1) to a cable (19), wherein the cable interface (18) is configured for laterally guiding the cable (19) in relation to the frame interior region (4).

12. Clamping device (1) according to one of the preceding claims, **characterized in that** the clamping device (1) is integral and composed of plastics material.

13. Clamping device (1) according to one of the preceding claims, **characterized in that** the clamping device (1) has a spring element (22), the latter being insert-moulded in plastics material.

14. Sensor cable (2) for electrically coupling an electrical contact with a diagnostics apparatus, having at least one monopolar cable (19) and at least one clamping device (1) for electrically coupling the sensor cable (2) to the electrical contact,
**characterized in that** the at least one clamping device (1) is configured as a clamping device (1) according to one of the preceding claims.

15. Sensor cable (2) according to Claim 14, **characterized in that** the cable (19) is configured so as to be multipolar, having a plurality of conductors (20), and **in that** the sensor cable has a plurality of clamping devices (1), wherein the clamping devices (1) are in each case electrically coupled to a different conductor (20) of the cable (19).

16. Sensor cable (2) according to Claim 14 or 15, **characterized in that** the at least one clamping device (1) is integrally cast on the cable (19).

## Revendications

1. Dispositif de serrage (1) dévolu au couplage électrique d'un câble de détection (2) avec un contact électrique, muni d'un cadre périphérique (3) élastiquement déformable, lequel cadre (3) comprend une zone d'encadrement (4) intérieure, une surface extérieure d'encadrement (5) tournée à l'opposé de la zone d'encadrement (4) intérieure, une surface intérieure d'encadrement (6) tournée vers ladite zone d'encadrement (4) intérieure, une première surface extrême d'encadrement (7) et une seconde surface extrême d'encadrement (8) pointant à l'opposé de ladite première surface extrême d'encadrement (7), un dispositif d'appui (9) retenu sur le cadre (3) et pénétrant dans ladite zone d'encadrement (4) intérieure, ainsi qu'un dispositif de contre-appui (10) implanté sur ledit cadre (3) et pourvu d'un élément de contre-butée (11), ledit dispositif d'appui (9) et ledit dispositif de contre-appui (10) étant réalisés de manière à former, en un emplacement de retenue du dispositif de serrage (1), un espace de réception conçu pour recevoir, ainsi que pour retenir le contact électrique, **caractérisé par le fait que** ledit dispositif de serrage (1) est réalisé de façon telle qu'il soit possible de provoquer par pression exercée de part et d'autre contre la surface extérieure d'encadrement (5), sur un premier axe (12) du cadre traversant la zone d'encadrement (4) intérieure, un mouvement relatif du dispositif de contre-appui (10) par rapport au dispositif d'appui (9), vers un premier emplacement de libération conçu pour libérer le contact électrique hors de l'espace de réception ; et qu'il soit possible de provoquer par pression exercée de part et d'autre depuis l'extérieur contre ladite surface extérieure d'encadrement (5), sur un second axe (13) du cadre différant dudit premier axe (12) dudit cadre et traversant ladite zone d'encadrement (4) intérieure, un mouvement relatif dudit dispositif de contre-appui (10) par rapport audit dispositif d'appui (9), vers un second emplacement de libération qui diffère dudit premier emplacement de libération et est conçu pour libérer ledit contact électrique hors dudit espace de réception.

2. Dispositif de serrage (1) selon la revendication 1,
**caractérisé par le fait que** ledit dispositif de serrage (1) est réalisé de façon telle qu'il soit possible de provoquer par pression exercée de part et d'autre contre la surface extérieure d'encadrement (5), sur le premier axe (12) du cadre, un mouvement du dispositif de contre-appui (10) éloignant ce dernier du dispositif d'appui (9).

3. Dispositif de serrage (1) selon la revendication 1 ou 2,
**caractérisé par le fait que** ledit dispositif de serrage (1) est réalisé de façon telle qu'il soit possible de provoquer par pression exercée de part et d'autre depuis l'extérieur contre la surface extérieure d'encadrement (5), sur le second axe (13) du cadre, un mouvement de l'élément de contre-butée (11) éloignant ce dernier dudit second axe (13) dudit cadre.

4. Dispositif de serrage (1) selon l'une des revendications précédentes, **caractérisé par le fait que** le dispositif de contre-appui (10) compte deux éléments de contre-butée (11), ledit dispositif de serrage (1) étant réalisé de façon telle qu'il soit possible de provoquer par pression exercée de part et d'autre contre la surface extérieure d'encadrement (5), sur le second axe (13) du cadre, un mouvement desdits éléments de contre-butée (11) éloignant ces derniers l'un de l'autre.

5. Dispositif de serrage (1) selon l'une des revendications précédentes, **caractérisé par le fait que** le cadre (3) comporte plusieurs zones d'articulation (14), lesquelles zones d'articulation (14) sont conçues pour céder élastiquement en réaction à la pression exercée, de part et d'autre, contre la surface extérieure d'encadrement (5) sur le premier axe (12) du cadre et/ou sur le second axe (13) dudit cadre.

6. Dispositif de serrage (1) selon l'une des revendications précédentes, **caractérisé par le fait que** le dispositif d'appui (9) est doté, au niveau d'une extrémité d'appui tournée vers le dispositif de contre-appui (10), d'une augette d'appui (15) conçue pour procurer un appui au contact électrique, en partie avec effet d'enlacement.

7. Dispositif de serrage (1) selon l'une des revendications précédentes, **caractérisé par le fait qu'**un contacteur électrique (16) est implanté sur le dispositif d'appui (9), en vue du couplage électrique dudit dispositif de serrage (1) avec le contact électrique.

8. Dispositif de serrage (1) selon la revendication 7,
**caractérisé par le fait que** le contacteur électrique (16) est réalisé et agencé de telle sorte qu'un couplage électrique du dispositif de serrage (1), avec le contact électrique, puisse avoir lieu par l'intermédiaire tant de la première surface extrême d'encadrement, que de la seconde surface extrême d'encadrement dudit dispositif de serrage (1).

9. Dispositif de serrage (1) selon l'une des revendications précédentes, **caractérisé par le fait que** la surface extérieure d'encadrement (5) est munie, au moins en partie, d'une face supérieure (17) prévenant le dérapage.

10. Dispositif de serrage (1) selon l'une des revendications précédentes,
**caractérisé par le fait qu'**au moins l'une des surfaces extrêmes d'encadrement (7, 8) est de réalisation(s) plane et/ou bombée.

11. Dispositif de serrage (1) selon l'une des revendications précédentes,
**caractérisé par le fait que** ledit dispositif de serrage (1) présente un interface de câblage (18) dédié au couplage électrique dudit dispositif de serrage (1) avec un câble (19), ledit interface de câblage (18) étant conçu en vue du guidage dudit câble (19) s'opérant latéralement par rapport à la zone d'encadrement (4) intérieure.

12. Dispositif de serrage (1) selon l'une des revendications précédentes,
**caractérisé par le fait que** ledit dispositif de serrage (1) est constitué d'une seule pièce, en une matière plastique.

13. Dispositif de serrage (1) selon l'une des revendications précédentes,
**caractérisé par le fait que** ledit dispositif de serrage (1) est pourvu d'un élément élastique (22) enrobé d'une matière plastique par injection.

14. Câble de détection (2) affecté au couplage électrique d'un contact électrique avec un appareil de diagnostics, comprenant un câble (19) au moins unipolaire, et au moins un dispositif de serrage (1) dévolu au couplage électrique dudit câble de détection (2) avec ledit contact électrique,
**caractérisé par le fait que** le dispositif de serrage (1), à présence minimale, est réalisé en tant que dispositif de serrage (1) conforme à l'une des revendications précédentes.

15. Câble de détection (2) selon la revendication 14,
**caractérisé par le fait que** le câble (19) est de réalisation multipolaire comprenant plusieurs conducteurs (20) ; et **par le fait que** le câble de détection comporte plusieurs dispositifs de serrage (1), lesquels dispositifs de serrage (1) sont couplés électriquement, à chaque fois, avec un conducteur différent (20) dudit câble (19).

16. Câble de détection (2) selon la revendication 14 ou 15,
**caractérisé par le fait que** le dispositif de serrage (1), à présence minimale, est coulé d'un seul tenant avec le câble (19).
